**Europäisches Patentamt**

**European Patent Office**

**Office européen des brevets**

(19)

(11) Numéro de publication : **0 569 496 B1**

(12)

# FASCICULE DE BREVET EUROPEEN

(45) Date de publication du fascicule du brevet :
**21.09.94 Bulletin 94/38**

(51) Int. Cl.$^5$ : **G01N 21/64**

(21) Numéro de dépôt : **92905184.5**

(22) Date de dépôt : **27.01.92**

(86) Numéro de dépôt international :
**PCT/FR92/00069**

(87) Numéro de publication internationale :
**WO 92/13264 06.08.92 Gazette 92/21**

(54) **PROCEDE DE MESURE DE LA LUMINESCENCE EMISE DANS UN DOSAGE PAR LUMINESCENCE.**

(30) Priorité : **28.01.91 FR 9100930**

(43) Date de publication de la demande :
**18.11.93 Bulletin 93/46**

(45) Mention de la délivrance du brevet :
**21.09.94 Bulletin 94/38**

(84) Etats contractants désignés :
**AT BE CH DE DK ES FR GB GR IT LI LU MC NL
SE**

(56) Documents cités :
**EP-A-02 668 81
EP-A-02 781 49
GB-A-22 158 38
GB-A-22 280 81
US-A- 4 100 416
US-A- 4 868 103
US-A- 4 876 190
US-A-43 419 57
US-A-45 421 04**

(73) Titulaire : **CIS BIO INTERNATIONAL
RN 306
F-91400 Saclay (FR)**

(72) Inventeur : **MABILE, Michel
46, avenue du Chesnay
F-78170 La Celle-Saint-Cloud (FR)**
Inventeur : **MATHIS, Gérard
17, impasse de la Capelle-des-Ladres
F-30200 Bagnols-sur-Cèze (FR)**
Inventeur : **JOLU, Etienne, Jean-Pierre
2, allée du Romarin
F-30200 Bagnols-sur-Cèze (FR)**
Inventeur : **POUYAT, Dominique
Rue Gérard-Philippe
F-30150 Roquemaure (FR)**
Inventeur : **DUMONT,Christophe
Ruelle Cuisy
F-60150 Thourotte (FR)**

(74) Mandataire : **Gillard, Marie-Louise et al
Cabinet Beau de Loménie
158, rue de l'Université
F-75340 Paris Cédex 07 (FR)**

EP 0 569 496 B1

## Description

L'invention a pour objet un procédé de mesure de la luminescence émise dans un dosage par luminescence permettant de corriger certaines perturbations dues au milieu de mesure.

L'utilisation de dosages immunologiques pour l'analyse qualitative et quantitative de composés dans des fluides biologiques est à l'heure actuelle largement répandue.

Parmi les techniques existantes, les dosages par fluorimétrie ont pris une importance croissante.

En effet, ils présentent un certain nombre d'avantages parmi lesquels la sensibilité, la rapidité de la mesure, la stabilité et l'inocuité des réactifs marqués par des composés fluorescents et le coût relativement réduit.

Il est connu que les méthodes de détection utilisant la fluorescence sont intrinsèquement très sensibles et pourraient permettre des limites de détection inférieures à celles atteintes par des dosages immunologiques utilisant des réactifs radiomarqués, en particulier par l'utilisation de sources lumineuses modulables laser (I. Wieder, Immunofluorescence and related staining techniques, 1978, Elsevier). Voir aussi GB-A-2215 838.

De nombreuses molécules fluorescentes utilisables comme traceur dans ce type de dosages ont été précédemment décrites; parmi celles-ci, on peut citer notamment les complexes de terre rare qui possèdent des propriétés intéressantes.

Par "traceur", on entend soit une molécule luminescente émettant une luminescence directe, soit une molécule luminescente capable d'induire une émission de luminescence, ladite molécule étant susceptible d'être couplée à l'un des réactifs du dosage, et l'émission de luminescence, directe ou induite, permettant la détection et/ou la détermination de l'analyte recherché.

L'utilisation de complexes particuliers, les cryptates de terre rare, est décrite par exemple dans les demandes EP O 321 353, 0 180 492, 0 232 348 ou la demande internationale WO 90/04791.

Ces cryptates de terre rare présentent l'avantage d'être très stables en milieu protéique et salin, cette propriété étant particulièrement importante dans le cas des dosages immunologiques homogènes.

La sensibilité de la mesure est néanmoins limitée par différents paramètres interférents parmi lesquels on peut citer :

- les propriétés spectroscopiques du milieu, et en particulier sa fluorescence intrinsèque, due notamment aux émissions parasites des molécules présentes dans le milieu de mesure et susceptibles d'être excitées et d'émettre à des longueurs d'ondes proches de celles du traceur fluorescent et/ou avec de fortes intensités ; son absorption, qui entraîne une perte de lumière excitatrice ; ses propriétés de diffusion de la lumière lorsque le milieu de mesure n'est pas limpide,
- l'extinction de la fluorescence émise ("quenching") par des inhibiteurs présents dans le milieu,
- la composition de l'appareillage, et notamment les réflexions parasites causées par l'appareillage,

L'ensemble de ces interférences affecte considérablement la sensibilité et la reproductibilité de la mesure.

Certains de ces problèmes ont déjà été résolus par diverses techniques.

En particulier, les méthodes de mesure de fluorescence en temps résolu permettent de remédier partiellement aux émissions parasites (bruit de fond ). Le principe de ces méthodes réside dans le fait qu'on effectue la mesure de la fluorescence émise par une molécule traceur ayant une durée de vie d'émission relativement longue, la mesure étant retardée dans le temps au-delà de la durée de vie d'émission des autres molécules présentes.

Il est dans ce cas nécessaire d'utiliser des molécules fluorescentes traceurs à durée de vie relativement longue telles que les chélates et les cryptates de terre rare.

Néanmoins, aucune solution satisfaisante n'a été apportée aux limitations dues aux propriétés spectroscopiques du milieu, et en particulier à son absorption.

En effet, parmi les techniques proposées pour éviter l'effet de filtre du milieu, aucune ne permet à la fois une mise en oeuvre aisée, peu coûteuse ainsi que l'obtention d'une grande sensibilité et d'une très bonne reproductibilité dans la mesure.

En particulier, la solution consistant à diluer fortement l'échantillon est défavorable à la sensibilité de la détection.

Par ailleurs, la mise en oeuvre d'un système à double faisceau d'excitation, entraîne l'utilisation d'appareillages coûteux et de cuvettes de mesure spéciales, difficiles à standardiser. De plus, la mesure systématique de l'absorption du milieu avant la mesure de la fluorescence de l'échantillon alourdit le procédé de dosage.

La demande EP 355 849 décrit une méthode et un appareillage automatique permettant de vérifier la fiabilité de la mesure de fluorescence d'un échantillon et de corriger cette mesure par rapport à une référence interne. Ceci nécessite préalablement à la mesure de l'échantillon à tester, d'effectuer une mesure avec 2 échantillons de référence, un "blanc" ne contenant que le milieu de mesure et l'autre contenant le milieu de mesure et le marqueur fluorescent.

2

EP 0 569 496 B1

La demande EP 91 126 concerne un fluorimètre permettant de mesurer, parallèlement à la fluorescence de l'échantillon, la transmission de celui-ci et les fluctuations de l'énergie d'excitation, afin de corriger la fluorescence mesurée. Ce système nécessite une cellule de mesure particulière laissant passer le faisceau d'excitation, car la mesure de la transmission doit s'effectuer dans l'alignement du faisceau d'excitation.

D'autres systèmes mettent en oeuvre un système de division du faisceau d'excitation, tel que décrit par exemple dans les demandes EP 174 722 et EP 241 268.

L'invention a donc pour objet un procédé de mesure de la luminescence émise dans un dosage par luminescence, caractérisé en ce qu'on met en oeuvre au moins un composé luminescent traceur et un composé luminescent utilisé comme référence interne qui , lorsqu'ils sont soumis à une même longueur d' onde d' excitation, sont susceptibles d' émettre soit par luminescence directe, soit par induction d'une émission de luminescence, à des longueurs d'onde différentes, respectivement $\lambda_2$ et $\lambda_1$, la luminescence du composé de référence reflétant la qualité optique du milieu, et en ce qu'on corrige la mesure de la luminescence émise par le composé traceur à la longueur d'onde $\lambda_2$ par la mesure de la luminescence émise par le composé de référence à la longueur d'onde $\lambda_1$.

Par "composé de référence" on entend soit une molécule luminescente émettant une luminescence directe, soit une molécule luminescente capable d'induire une émission de luminescence, ladite émission de luminescence, directe ou indirecte, n'étant pas perturbée par le système réactif du dosage.

Les composés luminescents utilisables dans le procédé de mesure selon l'invention peuvent soit émettre directement à leur longueur d'onde d'émission ou à une autre longueur d'onde comme par exemple dans le cas d'un déplacement de spectre lié au système réactif du dosage.

Les composés luminescents utilisables dans le procédé de mesure selon l'invention peuvent le cas échéant émettre indirectement en induisant une émission de luminescence, comme notamment dans le cas de méthodes homogènes par transfert d'énergie.

Avantageusement, les émissions de luminescence aux longueurs d'onde $\lambda_2$ et $\lambda_1$ sont détectées simultanément.

Le procédé selon l'invention , qui met en oeuvre un seul faisceau d'excitation, permet d'effectuer de manière aisée et fiable la mesure de la luminescence émise dans un dosage par luminescence sans nécessiter d'appareillage complexe, en éliminant les perturbations dues aux propriétés spectroscopiques du milieu de dosage.

De manière avantageuse, les longueurs d'onde d'émission du composé luminescent traceur et du composé luminescent de référence $\lambda_1$ et $\lambda_2$ seront différentes mais de préférence proches (en ayant une différence par exemple inférieure ou égale à 100 nm) de façon à ce que la perturbation de L'émission de luminescence due à l'absorption du milieu se produise de la même manière vis-à-vis de l'émission du composé traceur et de celle du composé de référence.

On notera que, avantageusement, le procédé selon la présente invention ne nécessite pas que l'échantillon à doser soit placé dans une cuve de mesure particulière.

L'émission de luminescence du composé de référence à longueur d'onde $\lambda_1$ permet de corriger la mesure réalisée la longueur d'onde $\lambda_2$. On peut effectuer la correction par exemple, en divisant cette dernière mesure par la mesure réalisée à la longueur d'onde $\lambda_1$. D'autres moyens de correction sont utilisables, tel que, par exemple, une méthode de correction intégrée à l'appareillage dans laquelle on fixe un taux de comptage sur le canal mesurant l'émission de luminescence du composé de référence à la longueur d'onde $\lambda_1$. Lorsque ce taux est atteint, on déclenche la fin de la mesure sur le canal mesurant l'émission de luminescence à la longueur d'onde $\lambda_2$. La valeur obtenue sur ce canal est de ce fait directement corrigée.

D'autres méthodes de correction connues de L'homme du métier sont également utilisables.

Dans un aspect préféré, l'invention a pour objet un procédé de mesure de la fluorescence émise dans un dosage par fluorométrie, caractérisé en ce qu'on met en oeuvre au moins un composé fluorescent traceur et un composé fluorescent utilisé comme référence interne qui, lorsqu'ils sont soumis à une même longueur d'onde d'excitation sont susceptibles d'émettre, soit par fluorescence directe, soit par induction d'une émission de fluorescence, à des longueurs d'onde différentes, respectivement $\lambda_2$ et $\lambda_1$, la fluorescence du composé de référence reflétant la qualité optique du milieu, et la mesure de la fluorescence émise par le composé traceur étant alors corrigée par la mesure de la fluorescence émise par le composé de référence.

Le procédé selon l'invention permet d'atteindre une sensibilité de mesure de l'ordre de la picomole/litre, alors que les phénomènes évoqués plus haut limitent habituellement la sensibilité du dosage , notamment lors de dosages homogènes en milieu sérique, à des concentrations en analyte de l'ordre de la micromole/litre.

Dans un aspect avantageux de l'invention, le composé luminescent traceur et le composé de référence sont un seul et même composé.

Cette première variante du procédé selon L'invention s'applique de préférence lors de l'utilisation d'un procédé homogène de détection et/ou de détermination par luminescence d'un analyte dans un milieu susceptible

3

de le contenir dans lequel la mesure de la luminescence émise représentative de la quantité d'analyte dans le milieu est effectuée à une longueur d'onde d'émission différente de celle du composé traceur.

Par exemple, ce cas se présente lorsque la luminescence émise représentative de l'analyte résulte d'un transfert d'énergie entre un composé luminescent donneur et un composé luminescent accepteur, ce dernier émettant à une longueur d'onde $\lambda_2$ alors que le composé donneur servant également de composé de référence émet à une longueur d'onde $\lambda_1$.

Notamment ce cas se présente également lorsque le composé traceur émet à des longueurs d'onde différentes $\lambda_1$ et $\lambda_2$ selon qu'il est, respectivement, non engagé ou engagé dans le système réactif du dosage.

Par "procédé homogène", on entend un procédé de dosage dans lequel la mesure ne nécessite pas la séparation préalable des constituants du dosage.

De manière surprenante, on a en effet trouvé que L'intensité du signal émis par le composé luminescent de référence à la longueur d'onde $\lambda_1$ est pratiquement constant. Le signal émis est donc fonction uniquement des propriétés optiques du milieu dans lequel on effectue le dosage et non de la quantité d'analyte, et peut servir de référence.

Dans le cas d'une émission de luminescence résultant d'un transfert d'énergie, le signal reflétant à la fois la quantité d'analyte à doser et les propriétés optiques du milieu de mesure est détecté à une longueur d'onde $\lambda_2$ et corrigé par la mesure réalisée à la longueur d'onde $\lambda_1$.

De préférence, on utilisera cette première variante du procédé selon l'invention dans un procédé homogène de détection et/ou de détermination par luminescence d' un analyte dans un milieu susceptible de le contenir à l'aide d'une méthode par excès consistant :

1) à ajouter, audit milieu contenant l'analyte recherché, un premier réactif constitué par au moins un récepteur dudit analyte, couplé avec un composé luminescent donneur,

2) à ajouter un second réactif constitué par un ou plusieurs autres récepteurs dudit analyte, ledit second réactif étant couplé avec un composé luminescent accepteur,

3) à faire incuber ledit milieu après chaque addition de réactifs ou après l'addition des deux réactifs,

4) à exciter le milieu résultant à la longueur d'onde d'excitation du composé luminescent donneur,

5) à mesurer le signal du composé luminescent donneur à une longueur d'onde $\lambda_1$, cette mesure servant de référence, et le signal résultant du transfert d'énergie à une longueur d'onde différente $\lambda_2$.

Dans un aspect avantageux, le premier réactif et le second réactif utilisés dans les procédés de détection et/ou de détermination par luminescence d'un analyte indiqués ci-dessus sont ajoutés simultanément au milieu contenant l'analyte recherché.

Dans un autre aspect de l'invention, on peut utiliser cette variante du procédé dans un procédé homogène de détection et/ou de détermination par luminescence d'un analyte dans un milieu susceptible de le contenir à l'aide d'une méthode par compétition consistant :

1) à ajouter, audit milieu contenant l'analyte recherché, un premier réactif constitué par au moins un récepteur dudit analyte, couplé avec un composé luminescent donneur,

2) à ajouter un second réactif constitué de l'analyte couplé avec un composé luminescent accepteur,

3) à faire incuber ledit milieu après chaque addition de réactifs ou après l'addition des deux réactifs,

4) à exciter le milieu résultant à la longueur d'onde d'excitation du composé luminescent donneur,

5) à mesurer le signal du composé luminescent donneur à une longueur d'onde $\lambda_1$, cette mesure servant de référence, et le signal résultant du transfert d'énergie à une longueur d'onde différente $\lambda_2$.

On peut également avantageusement utiliser cette variante du procédé dans un procédé homogène de détection et/ou de détermination par luminescence d'un analyte dans un milieu susceptible de le contenir à l'aide d'une méthode par compétition consistant :

1) à ajouter, audit milieu contenant l'analyte recherché, un premier réactif constitué par au moins un récepteur dudit analyte, couplé avec un composé luminescent accepteur,

2) à ajouter un second réactif constitué de l'analyte couplé avec un composé luminescent donneur,

3) à faire incuber ledit milieu après chaque addition de réactifs ou après l'addition des deux réactifs,

4) à exciter le milieu résultant à la longueur d'onde d'excitation du composé luminescent donneur,

5) à mesurer le signal du composé luminescent donneur à une longueur d'onde $\lambda_1$, cette mesure servant de référence, et le signal résultant du transfert d'énergie à une longueur d'onde différente $\lambda_2$.

Dans une autre utilisation avantageuse du procédé de l'invention, l'un au moins des récepteurs de l'analyte est fixé à un support solide.

Dans un autre aspect avantageux, le composé luminescent traceur et le composé de référence sont 2 composés distincts excitables à la même longueur d'onde, le composé de référence émettant à une longueur d'onde différente de celle utilisée pour mesurer la quantité d'analyte.

Cette seconde variante du procédé de l'invention est préférée en particulier dans l'utilisation d'un procédé homogène de détection et/ou de détermination par luminescence d'un analyte dans un milieu susceptible de le contenir dans lequel on utilise un réactif couplé avec un composé luminescent traceur et un réactif couplé avec un atome lourd ou des motifs contenant un atome lourd susceptible de moduler le signal du composé luminescent traceur. Un procédé de dosage de ce type est décrit dans la demande EP 232 348.

Dans ce cas, on utilisera en plus du composé luminescent traceur couplé au récepteur de l'analyte et émettant à une longueur d'onde $\lambda_2$, un autre composé luminescent libre servant de référence émettant à une longueur $\lambda_1$ dont le signal n'est pas modulé par l'effet d'atome lourd et reflète les propriétés optiques du milieu de mesure, ces 2 composés étant excités à la même longueur d'onde.

Avantageusement, cette seconde variante du procédé selon l'invention est utilisée dans un procédé homogène de détection et/ou de détermination par luminescence d'un analyte dans un milieu susceptible de le contenir consistant :

1) à ajouter audit milieu un premier réactif constitué d'un récepteur dudit analyte,

2) à ajouter un second réactif choisi parmi l'analyte ou au moins l'un de ses récepteurs, l'un des deux réactifs étant couplé avec un composé luminescent traceur et l'autre réactif comportant un atome lourd ou des motifs contenant un atome lourd, ainsi qu'un composé luminescent servant de référence interne,

3) à faire incuber le milieu résultant soit après l'addition de chaque réactif, soit après l'addition des deux réactifs,

4) à exciter le milieu résultant, et

5) à mesurer d'une part le signal émis par le composé luminescent traceur, ledit signal étant modulé par l'effet d'atome lourd à une longueur d'onde $\lambda_2$ , et d'autre part le signal émis par le composé de référence à une longueur $\lambda_1$.

Cette seconde variante du procédé dans laquelle le composé luminescent traceur et le composé de référence sont deux composés distincts est également avantageusement utilisable dans des procédés homogènes de détection et/ou de détermination d'un analyte dans un milieu susceptible de le contenir par des méthodes par excès ou par compétition mettant en jeu un système donneur/ accepteur, telles que décrites plus haut pour la première variante du procédé de l'invention.

Néanmoins, dans le cas de la seconde variante, il y a lieu d'ajouter un composé luminescent servant de référence interne au cours de l'une des étapes d'addition des réactifs.

On mesurera alors le signal du composé luminescent de référence à une longueur d'onde $\lambda_1$ et le signal résultant du transfert d'énergie à une longueur d'onde $\lambda_2$, cette mesure pouvant alors être corrigée par la mesure effectuée à $\lambda_1$.

Dans la présente description on définit par :

- "analyte" toute substance ou groupe de substances analogues à détecter et/ou déterminer ;
- "récepteur" toute substance capable de se fixer spécifiquement sur un site dudit analyte ;
- "atome lourd" un atome de nombre atomique élevé et dont la présence à proximité d'une molécule fluorescente est capable d'induire une augmentation du couplage spin-orbite de celle-ci. A titre d'exemples d'atomes lourds appropriés, on peut citer notamment les atomes d'halogènes, le mercure, le thallium, le plomb, l'argent ;
- "motif contenant au moins un atome lourd" toute substance chimique comportant naturellement au moins un atome lourd ou sur laquelle on peut fixer au moins un atome lourd.

Dans un aspect préféré, le milieu de mesure est un milieu biologique tel qu'un milieu sérique.

En tant que composés luminescents de référence et/ou utilisables comme composés traceurs, on utilisera avantageusement des composés fluorescents.

Notamment, on utilisera avantageusement un composé fluorescent tel qu'un chélate ou un cryptate de terre rare, en particulier un chélate ou un cryptate de terbium, europium, dysprosium, samarium ou néodynium. On utilisera de préférence un cryptate de terbium ou d'europium.

Dans les procédés de détection et/ou de détermination par fluorescence utilisant le procédé de mesure de l'invention, on choisira avantageusement un cryptate de terre rare décrit dans les demandes de brevets EP 180 492 et 321 353.

De préférence, on utilisera le cryptate de terbium : Tb trisbipyridine ou le cryptate d'europium : Eu trisbipyridine, tels que décrits dans la demande EP 180 492 ou les cryptates Eu trisbipyridine diamine et Tb trisbipyridine diamine décrits dans la demande EP 321 353.

Dans un aspect avantageux le composé fluorescent donneur est un cryptate d'europium et le composé fluorescent accepteur est choisi parmi l'allophycocyanine, l'allophycocyanine B, la C phycocyanine ou la R phycocyanine.

On peut également utiliser comme composé luminescent de référence ou comme composé traceur donneur, un composé phosphorescent tel que l'éosine ou l'erythrosine. Dans ce cas, on utilisera avantageusement un composé fluorescent accepteur choisi parmi les chlorophylles telles que celles citées dans les demandes EP 71 991 et EP 314 406, ou les porphyrines telles que citées dans la demande EP 71 991 ou encore les phtalocyanines telles que celles de la demande internationale WO 88 04777.

Dans le cas d'un dosage en milieu liquide utilisant des composés donneurs phosphorescents, la lecture sera effectuée soit sur un support solide, soit en ajoutant au milieu de mesure des molécules capteurs d'oxygène, ces techniques étant connues de l'homme du métier.

Les chlorophylles et les phtalocyanines peuvent également être utilisées comme composés accepteurs fluorescents en utilisant comme composé donneur un cryptate ou un chélate d'europium.

Dans un aspect préféré, le composé luminescent traceur et/ou le composé luminescent de référence ont une durée de vie supérieure à une microseconde.

Comme source de lumière permettant l'excitation des composés luminescents traceurs et de référence, on utilisera avantageusement une source de lumière modulable telle que celles décrites dans Lakowicz, Principles of fluorescent spectroscopy, Plenum Press, New-York, 1983, p. 96-100.

Le procédé de mesure de l'invention trouve en particulier une application importante dans les dosages immunologiques par fluorescence, aussi bien dans les méthodes de dosage dites par compétition que par excès, décrits dans l'art antérieur (Landon, Ann. Clin. Biochem, 1981, 18, 253 et E. SOINI et al, Clin. Chem. 1979, 25, 353).

En particulier, le procédé de mesure de l'invention peut être avantageusement utilisé dans les dosages immunologiques en milieu sérique.

Un aspect ultérieur de l'invention concerne un dispositif pour la mise en oeuvre du procédé de mesure selon l'invention.

Ce dispositif est caractérisé en ce qu'il comprend une source lumineuse d'excitation, des moyens pour collecter le faisceau lumineux émis à la suite de ladite excitation et des moyens pour permettre la mesure de la luminescence à deux longueurs d'onde distinctes.

Avantageusement, ce dispositif comprend également des moyens pour diviser le faisceau émis à la suite de l'excitation.

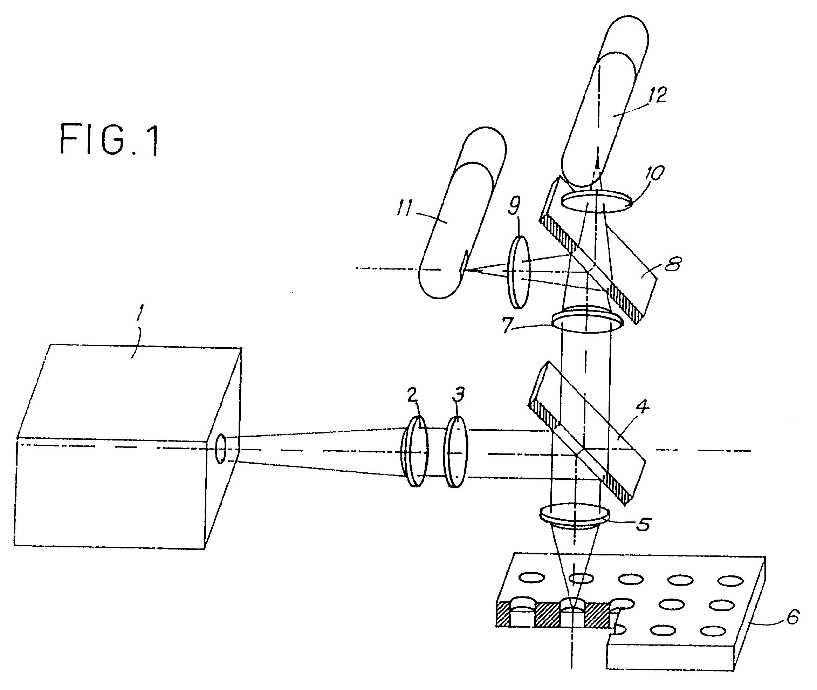

Un tel dispositif est illustré, à titre d'exemple, sur la figure 1.

La figure 1 représente :

- en 1, une source lumineuse d'excitation ;
- en 2, une lentille focalisant le faisceau excitant vers un filtre 3 sélectionnant la longueur d'onde souhaitée pour l'excitation ;
- un filtre dichroïque 4 placé à 45° réfléchissant les rayons ultraviolets et transmettant la lumière visible, qui réfléchit le faisceau excitant sur une lentille 5, celle-ci le focalisant sur la microplaque 6 contenant les échantillons ;
- en 7, une lentille collecte en combinaison avec la lentille 5 l'émission de luminescence résultant de l'excitation et la projete sur un filtre dichroïque 8 qui divise le faisceau lumineux émis ;
- les filtres 9 et 10 permettent de sélectionner les signaux émis par le composé de référence et le composé traceur, avant leur arrivée sur des photomultiplicateurs 11 et 12.

L'invention sera mieux comprise à l'aide des exemples ci-après qui ne présentent aucun caractère limitatif.

EXEMPLE 1 :

Correction de la fluorescence à 665 nm par détection d'un ajout de cryptate de terbium (trisbipyridine diamine ($Tb^{3+}$)) dans un dosage immunofluorescent homogène de la prolactine.

Cet exemple est réalisé sur la détection d'une gamme standard en antigène et sur le dosage de 5 échantillons sériques de concentration en prolactine inconnue.

METHODOLOGIE :

Dans ce dosage, on utilise comme composés fluorescents donneur le cryptate d'europium trisbipyridine diamine ($Eu^{3+}$) (traceur) et composé de référence le cryptate de terbium trisbipyridine diamine ($Tb^{3+}$) préparés comme décrit dans la demande EP 321 353 (exemples 3 et 4).

Comme composé fluorescent accepteur, on utilise l'allophycocyanine (Cyanotech, USA).

On utilise des anticorps monoclonaux anti-prolactine $E_1$ et 3D3 (CIS bio international, France) reconnaissant 2 épitopes distincts de la prolactine. La préparation des anticorps marqués par le cryptate d'europium-trisbipyridine diamine ($Eu^{3+}$) ou par l'allophycocyanine est décrite ci-dessous :

On utilise les abréviations suivantes :

| | |
|---|---|
| APC | = allophycocyanine |
| DTT | = dithiothreitol |
| EuTBP | = cryptate d'europium trisbipyridine diamine ($Eu^{3+}$) |

HSA = serum albumine humaine
IgG = immunoglobuline G
SPDP = N-succinimidyl 3(2-pyridyldithio)propionate
Sulfo-SMCC = sulfosuccinimidyl 4(n-maléimidométhyl)cyclohexane 1-carboxylate.

## 1) PREPARATION DES IgG $E_1$-APC

### a) Activation de l'APC par le sulfo-SMCC

L'APC (3 mg) commercialement fournie sous forme précipitée dans une solution à 60 % de sulfate d'ammonium, est centrifugée. Après élimination du surnageant, le culot est repris par 250 μl de tampon phosphate 100 mM, pH 7,0, puis filtré à 0,8 μm afin d'éliminer les éventuelles particules en suspension.

Le filtrat est purifié par chromatographie d'exclusion sur colonne G25 superfine (Pharmacia, Suède) dans le même tampon. La concentration d'APC éluée dans le volume d'exclusion est déterminée à 650 nm, en considérant un $\varepsilon_{650nm}$ de 731000 $M^{-1} CM^{-1}$.

L'activation de l'APC est réalisée en ajoutant une solution de sulfo-SMCC préparée extemporanément à raison de 6,9 mM dans un tampon phosphate 100 mM pH 7,0 et en laissant la réaction se produire pendant une heure, à température ambiante, sous agitation douce (rapport molaire de 15 à 75 sulfo-SMCC par APC). L'APC-maléimide est alors purifiée sur colonne G25 superfine en tampon phosphate 100 mM, EDTA 5 mM, pH 6,5 et conservée à 4°C avant couplage sur IgG 3D3.

### b) Activation des IgG $E_1$ par le SPDP

Simultanément, 5 mg d'IgG $E_1$ à raison de 10 mg/ml dans un tampon phosphate 100 mM, pH 7,0 sont activés par l'ajout d'une solution de SPDP (Pierce, USA) à raison de 6,4 mM dans du dioxane dans un rapport molaire de 4 à 16 SPDP par IgG $E_1$.

Après 35 min d'activation à température ambiante, l'IgG pyridine-2 thione est purifiée sur colonne G25 superfine dans un tampon phosphate 100 mM, EDTA 5mM, pH 6,5.

Les protéines sont concentrées et les groupes 2-pyridyl disulfides sont réduits par une solution de DTT (Sigma, USA) ayant une concentration finale de 19 mM pendant 15 min à température ambiante. Le DTT et la pyridine-2-thione sont éliminés par purification sur colonne G25 superfine en tampon phosphate 100 mM, EDTA 5mM, pH 6,5. La concentration en IgG-SH est déterminée à 280 nm avec un $\varepsilon_{280nm}$ de 210000 $M^{-1}cm^{-1}$.

### c) Conjugaison des IgG $E_1$-SH avec APC-maléimide

La fixation des groupements thiols sur les maléimides est réalisée en ajoutant 2,51 mg d'APC activées par mg d'IgG $E_1$-SH. Après 18 heures d'incubation à 4°C et à l'obscurité sous agitation douce, les fonctions thiols restées libres sont bloquées par l'addition d'une solution à 100 mM de N-méthyl maléimide (Sigma, USA) ayant une concentration finale de 20 mM pendant une heure à température ambiante.

Le milieu réactionnel est purifiée par gel filtration sur colonne TSK G3000SW semi-préparative (Beckmann, USA ) en tampon phosphate 100 mM pH 7,0.

Les concentrations en APC et en IgG $E_1$ du conjugué purifié, élué dans le premier pic, sont déterminées par les absorptions à 280 nm et à 650 nm, selon le calcul suivant :

$$[APC]_{Mole/l} = A_{650nM}/710000$$
$$[IgG]_{Mole/l} = (A_{280nm} - A'_{280nm})/210000$$

avec $A'_{280nm}$ étant la contribution à cette longueur d'onde de l'APC-maléimide.

De l'albumine sérique humaine (HSA) est rajoutée à concurrence de 1 g/l au conjugué qui est ensuite réparti en aliquotes puis congelé à -20°C.

## 2) PREPARATION DES CONJUGUES IgG 3D3 - Eu TBP

La préparation de IgG 3D3-SH est réalisée selon le protocole décrit plus haut pour les IgG $E_1$ mais avec un rapport molaire de 7,5 SPDP par IgG 3D3.

A 5 mg (5 $10^{-6}$moles) de Eu TBP est ajoutée une solution à 25 mM de sulfo-SMCC, en tampon phosphate 20 mM, diméthylformamide 10 % (v/v pH 7,0 dans une proportion de 2,5 moles d'activateur par mole de EuTBP.

Après 45 min d'activation à température ambiante, le milieu réactionnel est filtré à 0,8 μm afin d'éliminer le précipité éventuellement formé. Les produits réactionnels indésirables (sulfo-SMCC, N-hydroxysuccinimide, acide (N-maléimidométhyl) carboxylique) sont éliminés par chromatographie échangeuse d'ions sur colonne Mono Q (Pharmacia, Suède) en tampon phosphate 20 mM diméthylformamide 10 % (v/v), pH 7,0 sous choc de NaCl. La concentration en Eu TBP maléimide est déterminée à 307 nm avec un $\varepsilon_{307nm}$ de 25000 $M^{-1}cm^{-1}$ ainsi que le rapport A $_{307nm}$/A$_{280nm}$.

De façon similaire à celle décrite plus haut on fait réagir les fonctions maléimides avec les fonctions thiols fixés sur l'anticorps, dans des proportions molaires variant de 10 à 30 Eu TBP maléimide par IgG 3D3-SH.

Après 18 heures d'incubation à 4°C et blocage des groupements thiols (éventuellement restés libres) par N-méthylmaléimide, le Eu TBP non couplé est éliminé par dialyse en tampon phosphate 100 mM pH 7,0 à 4°C jusqu'à épuisement (plus de fluorescence dans les bains de dialyse).

Les caractéristiques du conjugué sont déterminées par ses absorptions à 307 nm et à 280 nm en utilisant les valeurs suivantes en tenant compte de l'absorption propre du cryptate déterminée par le rapport $A_{307nm}$/$A_{280nm}$.

Eu TBP-maléimide :

$$\varepsilon_{307nm} = 25000\ M^{-1}cm^{-1}$$

$A_{307nm}$/$A_{280nm}$ : déterminé expérimentalement.

IgG 3D3-SH :

$$\varepsilon_{280nm} = 210000\ M^{-1}cm^{-1}$$
$$\varepsilon_{307nm} = 0\ M^{-1}cm^{-1}$$

3) Dans des barrettes de 12 puits (Microstrip Labsystem Oy, Finlande) sont ajoutés successivement :

- 100 µl de standard (standards prolactine de la trousse ELSA-PRL, (CIS Bio international) en sérum de veau nouveau-né) ou 100 µl d'échantillon à doser (sérum humain),
- 100 µl d'anticorps monoclonal anti-prolactine 3D3, marqués au cryptate d'europium trisbipyridine diamine ($Eu^{3+}$), à la concentration de 0,5 g/ml d'anticorps en tampon phosphate 100 mM, NaF 150 mM, BSA (sérumalbumine bovine) 1 g/l, pH 7,0,
- 50 µl d'anticorps monoclonal anti-prolactine $E_1$ marqués à l'allophycocyanine à la concentration de 7 µg/ml d'anticorps en tampon phosphate 100 mM, NaF 150 mM, BSA 1g/l, pH 7,0,
- 50 µl de cryptate de terbium trisbipyridine diamine ($Tb^{3+}$) $10^{-7}$M en tampon phosphate 100 mM, NaF 150 mM, BSA 1 g/l, pH 7,0.

Après une heure d'incubation à 37°C, la fluorescence de chaque puits est mesurée selon deux protocoles :

Dans un premier protocole, la fluorescence à 665 nm est détectée en temps résolu, avec un délai de mesure de 50 µs et un temps d'intégration de 400 µs. La durée de la mesure est de 1 s. L'excitation est provoquée par une lampe flash, pulsée à 1000 Hz. La longueur d'onde d'excitation est centrée par un filtre interférentiel à 307 nm, maximum d'absorption du trisbipyridine diamine ($Eu^{3+}$) et trisbipyridine diamine ($Tb^{3+}$). L'intensité de fluorescence relevée, reflet du transfert d'énergie, est proportionnelle à la concentration en antigène prolactine présent dans le milieu d'incubation.

La mesure est réalisée au moyen d'un fluorimètre ARCUS (LKB, Suède) en utilisant un filtre interférentiel adapté à l'émission du composé fluorescent accepteur et du composé de référence.

On peut également réaliser la mesure en une seule étape en utilisant le fluorimètre prototype décrit dans l'exemple 2. Dans ce cas on utilisera pour la mesure une microplaque blanche de 96 puits (Dynatech, USA).

La courbe standard $FLUO_{665nm}$ = f ([Prolactine]) est tracée et la concentration des 5 échantillons est mesurée.

Les résultats sont rapportés dans le tableau I ci-après :

EP 0 569 496 B1

## TABLEAU I

| Standard ELSA-PRL | [Prolactine] $\mu$UI/ml |
|---|---|
| $STD_0$ | 0 |
| $STD_1$ | 165 |
| $STD_2$ | 300 |
| $STD_3$ | 920 |
| $STD_4$ | 3100 |
| $STD_5$ | 6500 |

| Sérum à doser | [Prolactine] ($FLUO_{665}$) $\mu$UI/ml |
|---|---|
| Ech1 | 141 |
| Ech2 | 951 |
| Ech3 | 113 |
| Ech4 | 287 |
| Ech5 | 699 |

Dans un second protocole la fluorescence à 665nm est mesurée de la même façon que décrite précédemment. Mais une seconde mesure du même puits est réalisée à 545nm après une seconde excitation, sous les mêmes paramètres temporels de lecture (délai, temps d'intégration et durée de la mesure). L'émission de fluorescence ainsi mesurée en temps résolu à 545 nm est caractéristique de l'émission du trisbipyridine diamine ($Tb^{3+}$) utilisé comme référence interne et reflète les propriétés optiques du milieu à doser.

La courbe standard $FLUO_{665nm}/FLUO_{545nm}$ = f([Prolactine]) est tracée et la concentration des 5 échantillons est mesurée.

Les résultats sont rapportés dans le tableau II ci-après:

9

## TABLEAU II

| Standard ELSA-PRL | [Prolactine] $\mu UI/ml$ |
|---|---|
| $STD_0$ | 0 |
| $STD_1$ | 165 |
| $STD_2$ | 300 |
| $STD_3$ | 920 |
| $STD_4$ | 3100 |
| $STD_5$ | 6500 |

| Sérum à doser | [Prolactine] $(FLUO_{665}/FLUO_{545})$ $\mu UI/ml$ |
|---|---|
| Ech1 | 580 |
| Ech2 | 428 |
| Ech3 | 417 |
| Ech4 | 71 |
| Ech5 | 2044 |

Enfin, en temps que référence, les 5 échantillons sont également dosés par le kit de dosage radio-immunométrique ELSA-PRL (CIS bio international, France).

Les comparaisons des résultats de dosages effectués sur les cinq échantillons sont rassemblés dans le tableau III ci-après:

## TABLEAU III

[Prolactine] $\mu UI/ml$

| | ELSA-PRL | $FLUO_{665}$ | $FLUO_{665}/FLUO_{545}$ |
|---|---|---|---|
| Ech1 | 612 | 141 | 580 |
| Ech2 | 395 | 951 | 428 |
| Ech3 | 425 | 113 | 417 |
| Ech4 | 59 | 287 | 71 |
| Ech5 | 1870 | 699 | 2044 |

Ces résultats montrent que les mesures des concentrations des échantillons de prolactine par la méthode comprenant une correction de l'intensité de fluorescence ($FLUO_{665}/FLUO_{545}$) sont en bonne corrélation avec les résultats obtenus à l'aide du dosage radio-immunométrique ELSA-PRL, alors que ce n'est pas le cas lorsque la concentration est mesurée uniquement par l'intensité de fluorescence à 665nm sans correction ($FLUO_{665}$).

EXEMPLE 2 :

Correction de la fluorescence à 665nm par détection de la fluorescence du conjugué anticorps-cryptate à 620nm dans un dosage immunofluorescent homogène de la prolactine.

La mesure de la fluorescence est réalisée à l'aide d'un fluorimètre prototype, qui est décrit ci-après :

Un Laser N2 VSL 337 (LSI, USA) est utilisé comme source d'excitation (longueur d'onde à 337 nm). La durée des pulsations est spécifiée à 3 nanosecondes et est répétée sous une fréquence de 10 Hertz. Le faisceau passe à travers un filtre (CORNING) afin d'éliminer toute lumière parasite à l'excitation autre que 337 nm.

Après être rentré dans la chambre de mesure, le faisceau est réfléchi par un filtre dichroïque, placé à 45 degrés, qui a la propriété de réfléchir les ultraviolets et de pouvoir transmettre la lumière visible.

Le faisceau réfléchi par lefiltre dichroïque est focalisé sur le puits à mesurer d'une microplaque par une lentille en silice fondue. L'émission de fluorescence est collectée selon un angle solide de 20 degrés, collimatée par la même lentille, et passe directement à travers le filtre dichroïque (fluorescence en lumière visible).

Un filtre interférentiel, de caractéristiques définies selon la longueur d'onde de fluorescence à détecter, permet de se débarrasser des lumières pouvant parasiter le signal, dont l'intensité est ensuite mesurée par un photomultiplicateur (HAMAMATSU R2949).

Le compteur de photons utilisé est un SR-400 (STANFORD RESEARCH SYSTEMS), dont les opérations et la synchronisation avec le laser sont contrôlées par un ordinateur de type IBM PC-AT via une sortie RS 232 . Les pulsations provenant du photomultiplicateur sont enregistrées pendant une fenêtre de temps ($t_g$) et après un délai ($t_d$) déterminés à condition qu'elles soient supérieures à un niveau discriminant sélectionné par le compteur de photons afin d'optimiser le rapport signal/bruit duphotomultiplicateur.

Une table X-Y, pilotée par l'IBM PC-AT, permet les différents positionnements de la microplaque de mesure par des moteurs pas à pas, incluant les manoeuvres de chargement, de positionnement sous le faisceau excitant, de lecture automatique en séquentiel des 96 puits, et de sortie.

I. Variation du signal d'un conjugué cryptate à 620nm en fonction du sérum.

Dans une microplaque blanche de 96 puits (Dynatech, USA) on introduit :
- 100 µl de sérum à tester
- 200 µl d'un conjugué anticorps anti-prolactine-cryptate d'europium à la concentration de 0,5 µg/ml d'anticorps en tampon phosphate 100 mM pH 7,5, HSA 1 g/l, NaF 150 mM.

Le cryptate d'europium utilisé est le trisbipyridine diamine ($Eu^{3+}$) dont la préparation est décrite dans la demande EP 321 353 (exemples 3 et 4).

L'anticorps anti-prolactine utilisé est l'anticorps 3D3 (CIS bio international, France).

Le conjugué anticorps 3D3-cryptate d'europium est préparé comme décrit dans l'exemple 1.

Après 1 heure d'incubation à 37°C, la fluorescence est détectée à 620nm, en temps résolu, avec un délai de mesure de 50 µs et un temps d'intégration de 400 µs. La durée de mesure est de 1 s.

La densité optique du sérum est mesurée à 310nm à L'aide d'un spectrophotomètre Lambda 15 PERKIN ELMER (UK).

Les résultats sont rapportés dans le tableau IV ci-après :

### TABLEAU IV

| Sérum | Do à 310 nm | Signal à 620 nm |
|---|---|---|
| 1 | 0,66 | 25 693 |
| 2 | 3,3 | 7 754 |
| 3 | 0,33 | 33 156 |
| 4 | 0,90 | 24 959 |

Ces résultats montrent que le signal mesuré varie fortement en fonction de la densité optique du sérum.

Dans ces conditions, il est impossible de réaliser un dosage fiable car le résultat ne dépend pas seulement de la quantité d'analyte dans le sérum mais également de la qualité optique du sérum.

II. Correction du signal de l'accepteur.

EP 0 569 496 B1

On réalise ensuite un immunoessai en ajoutant successivement dans la microplaque :
- 100 µl de sérum
- 100 µl de conjugué anticorps monoclonal anti-prolactine 3D3-cryptate d'europium trisbipyridine diamine ($Eu^{3+}$) à la concentration de 0,5 µl/ml d'anticorps dans le tampon phosphate ci-dessus,
- 100 µl de conjugué anticorps monoclonal anti-prolactine $E_1$-allophycocyanine à la concentration de 3,5 µg/ml d'anticorps dans le même tampon.

Ce conjugué anticorps $E_1$ (CIS bio international France)-allophycocyanine (Cyanotech, USA) est préparé comme décrit dans l'exemple 1.

La mesure est réalisée à 665nm et la valeur mesurée est corrigée en divisant cette valeur par la valeur de la fluorescence mesurée à 620nm qui reflète les propriétés optiques du milieu.

Les valeurs de concentrations déterminées à l'aide d'une courbe étalon comme indiqué dans L'exemple 1, avec ou sans correction, sont comparées avec les valeurs obtenues en utilisant comme référence le kit de dosage radio-immunométrique ELSA-PRL (CIS bio international France).

Les résultats sont rapportés dans le tableau V ci-après dans lequel figurent les valeurs des concentrations en prolactine exprimées en µU/ml déterminées respectivement par le dosage ELSA-PRL (ELSA) et par la mesure de la fluorescence à 665nm non corrigée ($FIA_{665}$) et corrigée ($FIA_{665/620}$), ainsi que la valeur de la fluorescence mesurée pour les différents échantillons à 620nm ($FLUO_{620}$) exprimée en coups par seconde.

TABLEAU V

| Echantillon n° | ELSA | $FIA_{665/620}$ | $FIA_{665}$ | $FLUO_{620}$ |
|---|---|---|---|---|
| 1 | 358 | 394 | 195 | 42148 |
| 2 | 530 | 592 | 91 | 28272 |
| 3 | 65 | 92 | 18 | 40495 |
| 4 | 153 | 178 | 94 | 43363 |
| 5 | 251 | 289 | 86,7 | 38007 |
| 6 | 161 | 146 | 86,4 | 44531 |
| 7 | 179 | 189 | 77 | 40873 |
| 8 | 285 | 297 | 161 | 41403 |
| 9 | 310 | 330 | 106 | 35214 |
| 10 | 2744 | 3427 | 1470 | 31670 |
| 11 | 196 | 153 | 15 | 36551 |
| 12 | 244 | 198 | 55 | 38134 |
| 13 | 447 | 477 | 300 | 40340 |
| 14 | 251 | 234 | 350 | 58615 |

Ces résultats confirment la variabilité du signal mesuré en fonction de l'échantillon de sérum dosé.

En effet, étant donné que le conjugué anticorps-cryptate se trouve en excès dans le dosage par rapport à l'analyte recherché, le signal mesuré à 620nm (qui correspond au conjugué non engagé dans le complexe) devrait être constant.

De plus, on remarque que les valeurs de concentrations en prolactine déterminées à partir de la mesure du signal à 665nm ($FIA_{665}$) ne correspondent pas aux valeurs obtenues par le dosage de référence, alors que les valeurs obtenues par la mesure corrigée ($FIA_{665/620}$) sont en bonne corrélation avec celles-ci.

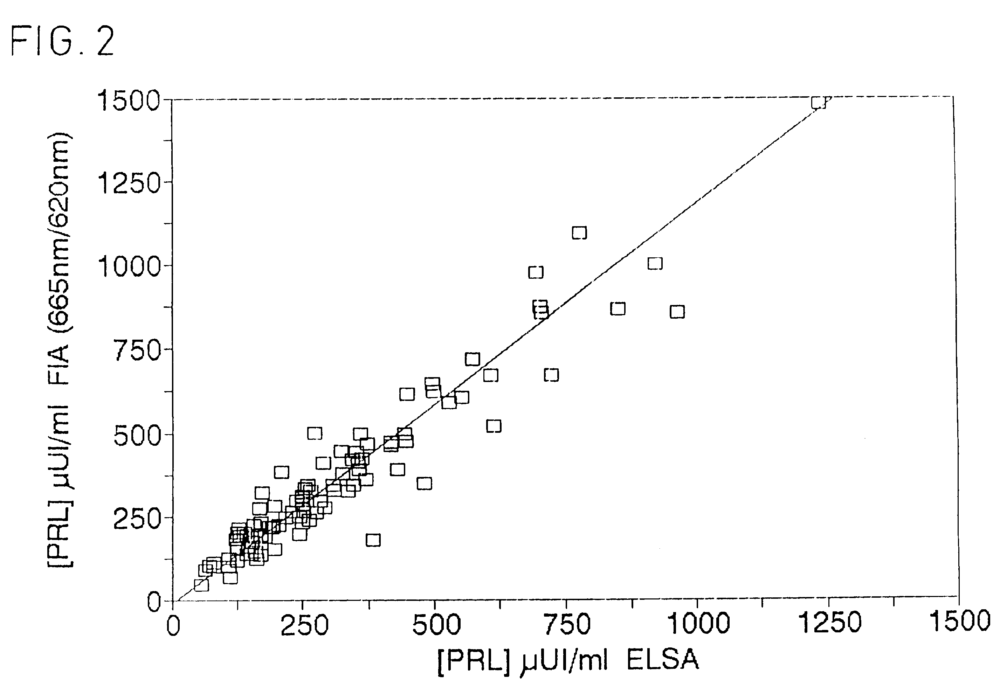

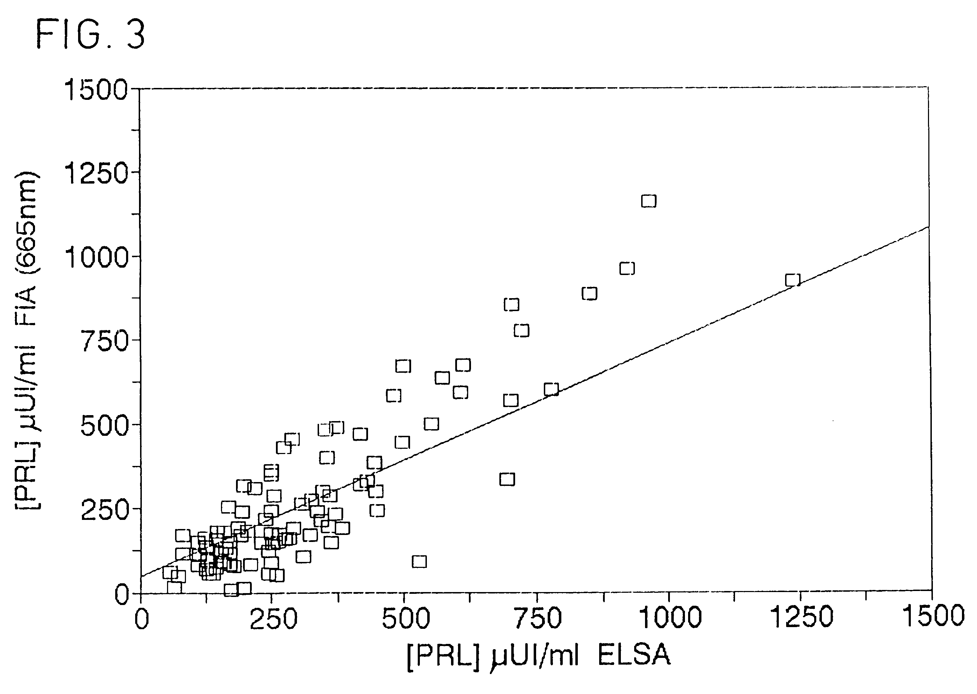

Cette corrélation est illustrée par les courbes des figures 2 et 3, sur lesquelles sont représentées en abscisse la concentration en prolactine déterminée par le test ELSA-PRL en UI/ml et en ordonnée, respectivement, la concentration déterminée par la mesure corrigée $FIA_{665/620}$ (figure 2), ou par la mesure non corrigée $FIA_{665}$ (figure 3), exprimée en UI/ml.

12

Les résultats obtenus sur 97 sera, montrent que la corrélation n'est obtenue qu'avec la correction du signal mesuré à 665nm.

## Revendications

1. Procédé de mesure de la luminescence émise dans un dosage par luminescence, caractérisé en ce qu'on met en oeuvre au moins un composé luminescent traceur et un composé luminescent utilisé comme référence interne qui, lorsqu'ils sont soumis à une même longueur d'onde d'excitation, sont susceptibles d'émettre soit par luminescence directe, soit par induction d'une émission de luminescence, à des longueurs d'onde différentes, respectivement $\lambda_2$ et $\lambda_1$, et en ce qu'on corrige la mesure de la luminescence émise par le composé traceur à la longueur d'onde $\lambda_2$ par la mesure de la luminescence émise par le composé de référence à la longueur d'onde $\lambda_1$.

2. Procédé selon la revendication 1, caractérisé en ce que le composé luminescent traceur et le composé de référence sont un seul et même composé.

3. Procédé selon la revendication 1, caractérisé en ce que le composé luminescent traceur et le composé de référence sont distincts.

4. Procédé selon l'une quelconque des revendications 1 à 3, caractérisé en ce que le composé luminescent traceur et/ou le composé de référence sont des composés fluorescents.

5. Procédé selon l'une quelconque des revendications 1 à 4, caractérisé en ce que le composé luminescent traceur et/ou le composé de référence sont des chélates ou des cryptates de terre rare.

6. Procédé selon l'une quelconque des revendications 1 à 5, caractérisé en ce que le composé luminescent traceur et/ou le composé de référence ont une durée de vie supérieure à une microseconde.

7. Procédé selon l'une quelconque des revendications 1 à 6 , caractérisé en ce qu'on effectue simultanément la mesure de la luminescence émise par le composé de référence et par le composé traceur, respectivement, aux longueurs d'onde $\lambda_1$ et $\lambda_2$.

8. Utilisation du procédé selon la revendication 1 dans un procédé homogène de détection et/ou de détermination d'un analyte dans un milieu susceptible de le contenir.

9. Utilisation selon la revendication 8, dans un procédé homogène de détection et/ou de détermination d'un analyte dans un milieu susceptible de le contenir à l'aide d'une méthode par excès consistant :
   1) à ajouter, audit milieu contenant l'analyte recherché, un premier réactif constitué par au moins un récepteur dudit analyte, couplé avec un composé luminescent donneur,
   2) à ajouter un second réactif constitué par un ou plusieurs autres récepteurs dudit analyte, ledit second réactif étant couplé avec un composé luminescent accepteur,
   3) à faire incuber ledit milieu après chaque addition de réactifs ou après l'addition des deux réactifs,
   4) à exciter le milieu résultant à la longueur d'onde d'excitation du composé luminescent donneur,
   5) à mesurer le signal du composé luminescent donneur à une longueur d'onde $\lambda_1$, cette mesure servant de référence, et le signal résultant du transfert d'énergie à une longueur d'onde $\lambda_2$.

10. Utilisation selon la revendication 8, dans un procédé homogène de détection et/ou de détermination d'un analyte dans un milieu susceptible de le contenir à l'aide d'une méthode par compétition consistant :
    1) à ajouter, audit milieu contenant l'analyte recherché, un premier réactif constitué par un récepteur dudit analyte, couplé avec un composé luminescent donneur,
    2) à ajouter un second réactif constitué de l'analyte couplé avec un composé luminescent accepteur,
    3) à faire incuber ledit milieu après chaque addition de réactifs ou après l'addition des deux réactifs,
    4) à exciter le milieu résultant à la longueur d'onde d'excitation du composé luminescent donneur,
    5) à mesurer le signal du composé luminescent donneur à une longueur d'onde $\lambda_1$, cette mesure servant de référence, et le signal résultant du transfert d'énergie à une longueur d'onde différente $\lambda_2$.

11. Utilisation selon la revendication 8, dans un procédé homogène de détection et/ou de détermination d'un analyte dans un milieu susceptible de le contenir à l'aide d'une méthode par compétition consistant :

1) à ajouter, audit milieu contenant l'analyte recherché, un premier réactif constitué par un récepteur dudit analyte, ledit récepteur étant couplé avec un composé luminescent accepteur,

2) à ajouter un second réactif constitué de l'analyte couplé avec un composé luminescent donneur,

3) à faire incuber ledit milieu après chaque addition de réactifs ou après l'addition des deux réactifs,

4) à exciter le milieu résultant à la longueur d'onde d'excitation du composé luminescent donneur,

5) à mesurer le signal du composé luminescent donneur à une longueur d'onde $\lambda_1$, cette mesure servant de référence, et le signal résultant du transfert d'énergie à une longueur d'onde différente $\lambda_2$.

12. Utilisation selon la revendication 8, dans un procédé homogène de détection et/ou de détermination d'un analyte dans un milieu susceptible de le contenir à l'aide d'une méthode consistant:

1) à ajouter audit milieu un premier réactif constitué d'un récepteur dudit analyte,

2) à ajouter un second réactif choisi parmi l'analyte ou au moins l'un de ses récepteurs, l'un des deux réactifs étant couplé avec un composé luminescent traceur et l'autre réactif comportant un atome lourd ou des motifs contenant un atome lourd, ainsi qu'un composé luminescent servant de référence interne,

3) à faire incuber le milieu résultant soit après l'addition de chaque réactif soit après l'addition des deux réactifs,

4) à exciter le milieu résultant, et

5) à mesurer d'une part le signal émis par le composé luminescent traceur, ledit signal étant modulé par l'effet d'atome lourd à une longueur d'onde $\lambda_2$ , et d'autre part le signal émis par le composé de référence à une longueur $\lambda_1$.

13. Utilisation selon l'une quelconque des revendications 9 à 12, caractérisée en ce qu'on effectue simultanément la mesure de la luminescence émise par le composé de référence et par le composé traceur, respectivement, aux longueurs d'onde $\lambda$1 et $\lambda$2.

14. Utilisation selon l'une quelconque des revendications 9 à 11, caractérisée en ce qu'au moins l'un des récepteurs de l'analyte est fixé à un support solide.

15. Procédé selon l'une quelconque des revendications 1 à 7, caractérisé en ce que la correction de la mesure de la luminescence émise par le composé traceur à la longueur d'onde $\lambda_2$ consiste à fixer un taux de comptage sur le canal mesurant l'émission de luminescence du composé de référence à la longueur d'onde $\lambda_1$, puis, lorsque ce taux est atteint, à déclencher la fin de la mesure sur le canal mesurant l'émission de luminescence à la longueur d'onde $\lambda_2$.

## Patentansprüche

1. Verfahren zur Lumineszenzmessung in einer Lumineszenzbestimmung, dadurch gekennzeichnet, daß wenigstens eine lumineszierende Tracerverbindung und eine als interne Referenz verwendete lumineszierende Verbindung eingesetzt werden, die, wenn sie einer gleichen Erregungswellenlänge ausgesetzt sind, geeignet sind, entweder durch direkte Lumineszenz oder durch Induktion einer Lumineszenzemission mit verschiedenen Wellenlängen $\lambda_2$ bzw. $\lambda_1$ zu emittieren, und daß die Messung der von der Tracerverbindung mit der Wellenlänge $\lambda_2$ emittierten Lumineszenz durch die Messung der von der Referenzverbindung mit der Wellenlänge $\lambda_1$ emittierten Lumineszenz korrigiert wird.

2. Verfahren nach Anspruch 1, dadurch gekennzeichnet, daß die lumineszierende Tracerverbindung und die Referenzverbindung ein und dieselbe Verbindung sind.

3. Verfahren nach Anspruch 1, dadurch gekennzeichnet, daß die lumineszierende Tracerverbindung und die Referenzverbindung unterschiedlich sind.

4. Verfahren nach einem der Ansprüche 1 bis 3, dadurch gekennzeichnet, daß die lumineszierende Tracerverbindung und/oder die Referenzverbindung fluoreszierende Verbindungen sind.

5. Verfahren nach einem der Ansprüche 1 bis 4, dadurch gekennzeichnet, daß die lumineszierende Tracerverbindung und/oder die Referenzverbindung Chelate oder Kryptate seltener Erden sind.

6. Verfahren nach einem der Ansprüche 1 bis 5, dadurch gekennzeichnet, daß die lumineszierende Tracerverbindung und/oder die Referenzverbindung eine Lebensdauer von mehr als einer Mikrosekunde.

**7.** Verfahren nach einem der Ansprüche 1 bis 6, dadurch gekennzeichnet, daß die Messung der von der Referenzverbindung bzw. von der Tracerverbindung emittierten Lumineszenz mit den Wellenlängen $\lambda_2$ bzw. $\lambda_1$ gleichzeitig erfolgt.

**8.** Anwendung des Verfahrens nach Anspruch 1 in einem homogenen Verfahren zur Detektion und/oder Ermittlung eines Analyten in einer Umgebung, die geeignet ist, ihn zu enthalten.

**9.** Anwendung nach Anspruch 8 in einem homogenen Verfahren zur Detektion und/oder Ermittlung eines Analyten in einer Umgebung, die geeignet ist, ihn zu enthalten, mit Hilfe einer Überschußmethode, die umfaßt:

1) Zugeben eines ersten Reaktanten in die den gesuchten Analyten beinhaltende Umgebung, der aus wenigstens einem Rezeptor des Analyten besteht und mit einer lumineszierenden Donorverbindung gekoppelt ist,

2) Zugeben eines zweiten Reaktanten, der aus einem oder mehreren Rezeptoren des Analyten besteht, wobei der zweite Reaktant mit einer lumineszierenden Akzeptorverbindung gekoppelt ist,

3) Ruhenlassen der Umgebung nach jeder Zugabe eines Reaktanten oder nach der Zugabe beider Reaktanten,

4) Anregen der erhaltenen Umgebung mit der Anregungswellenlänge der lumineszierenden Donorverbindung,

5) Messen des Signals der lumineszierenden Donorverbindung mit einer Wellenlänge $\lambda_1$, wobei diese Messung als Referenz dient, und des aus dem Energieübertrag resultierenden Signals mit einer Wellenlänge $\lambda_2$.

**10.** Anwendung nach Anspruch 8 in einem homogenen Verfahren zur Detektion und/oder Ermittlung eines Analyten in einer Umgebung, die geeignet ist, ihn zu enthalten, mit Hilfe einer Wettbewerbsmethode, die umfaßt:

1) Zugeben eines ersten Reaktanten in die den gesuchten Analyten beinhaltende Umgebung, der aus einem Rezeptor des Analyten besteht und mit einer lumineszierenden Donorverbindung gekoppelt ist,

2) Zugeben eines zweiten Reaktanten, der aus dem Analyten besteht und mit einer lumineszierenden Akzeptorverbindung gekoppelt ist,

3) Ruhenlassen der Umgebung nach jeder Zugabe eines Reaktanten oder nach der Zugabe beider Reaktanten,

4) Anregen der erhaltenen Umgebung mit der Anregungswellenlänge der lumineszierenden Donorverbindung,

5) Messen des Signals der lumineszierenden Donorverbindung mit einer Wellenlänge $\lambda_1$, wobei diese Messung als Referenz dient, und des aus dem Energieübertrag resultierenden Signals mit einer anderen Wellenlänge $\lambda_2$.

**11.** Anwendung nach Anspruch 8 in einem homogenen Verfahren zur Detektion und/oder Ermittlung eines Analyten in einer Umgebung, die geeignet ist, ihn zu enthalten, mit Hilfe einer Wettbewerbsmethode, die umfaßt:

1) Zugeben eines ersten Reaktanten in die den gesuchten Analyten beinhaltende Umgebung, der aus einem Rezeptor des Analyten besteht, wobei der Rezeptor mit einer lumineszierenden Akzeptorverbindung gekoppelt ist,

2) Zugeben eines zweiten Reaktanten, der aus dem Analyten besteht und mit einer lumineszierenden Donorverbindung gekoppelt ist,

3) Ruhenlassen der Umgebung nach jeder Zugabe eines Reaktanten oder nach der Zugabe beider Reaktanten,

4) Anregen der erhaltenen Umgebung mit der Anregungswellenlänge der lumineszierenden Donorverbindung,

5) Messen des Signals der lumineszierenden Donorverbindung mit einer Wellenlänge $\lambda_1$, wobei diese Messung als Referenz dient, und des aus dem Energieübertrag resultierenden Signals mit einer anderen Wellenlänge $\lambda_2$.

**12.** Anwendung nach Anspruch 8 in einem homogenen Verfahren zur Detektion und/oder Ermittlung eines Analyten in einer Umgebung, die geeignet ist, ihn zu enthalten, mit Hilfe einer Methode, die umfaßt:

1) Zugeben eines ersten Reaktanten in die Umgebung, der aus einem Rezeptor des Analyten besteht,

2) Zugeben eines zweiten Reaktanten, der aus dem Analyten oder wenigstens einem seiner Rezeptoren ausgesucht ist, wobei einer der beiden Reaktanten mit einer lumineszierenden Tracerverbindung gekoppelt ist und der andere Reaktant ein schweres Atom oder ein schweres Atom enthaltende Strukturen aufweist, als auch einer lumineszierenden Verbindung, die als interne Referenz dient,

3) Ruhenlassen der erhaltenen Umgebung entweder nach der Zugabe jedes Reaktanten oder nach der Zugabe beider Reaktanten,

4) Anregen der Umgebung, und

5) Messen einerseits des von der lumineszierenden Tracerverbindung emittierten Signals, das durch die Einwirkung des schweren Atoms mit einer Wellenlänge $\lambda_2$ moduliert ist, und andererseits des von der Referenzverbindung mit der Wellenlänge $\lambda_1$ emittierten Signals.

13. Anwendung nach einem der Ansprüche 9 bis 12, dadurch gekennzeichnet, daß die Messung der von der Referenzverbindung bzw. von der Tracerverbindung emittierten Lumineszenz mit den Wellenlängen $\lambda_2$ bzw. $\lambda_1$ gleichzeitig erfolgt.

14. Anwendung nach einem der Ansprüche 9 bis 11, dadurch gekennzeichnet, daß wenigstens einer der Rezeptoren des Analyten an einem festen Träger befestigt ist.

15. Verfahren nach einem der Ansprüche 1 bis 7, dadurch gekennzeichnet, daß die Korrektur der Messung der von der Tracerverbindung mit der Wellenlänge $\lambda_2$ emittierten Lumineszenz darin besteht, auf dem Kanal, der die Lumineszenzemission der Referenzverbindung mit der Wellenlänge $\lambda_1$ mißt, eine Zählrate festzulegen und dann, wenn diese Rate erreicht ist, das Ende der Messung auf dem Kanal auszulösen, der die Lumineszenzemission mit der Wellenlänge $\lambda_2$ mißt.

## Claims

1. Method of measuring the luminescence emitted in a luminescent assay, characterized in that at least one luminescent tracer compound and a luminescent compound used as an internal reference are employed, which, when exposed to the same excitation wavelength, are capable of emitting at different wavelengths, $\lambda_2$ and $\lambda_1$ respectively, either by direct luminescence or by the induction of a luminescent emission, and in that the measurement of the luminescence emitted by the tracer compound at wavelength $\lambda_2$ is corrected on the basis of the measurement of the luminescence emitted by the reference compound at wavelength $\lambda_1$.

2. Method according to claim 1, characterized in that the luminescent tracer compound and the reference compound are one and the same compound.

3. Method according to claim 1, characterized in that the luminescent tracer compound and the reference compound are different.

4. Method according to any one of claims 1 to 3, characterized in that the luminescent tracer compound and/or the reference compound are fluorescent compounds.

5. Method according to any one of claims 1 to 4, characterized in that the luminescent tracer compound and/or the reference compound are rare earth chelates or cryptates.

6. Method according to any one of claims 1 to 5, characterized in that the luminescent tracer compound and/or the reference compound have a lifetime of more than one microsecond.

7. Method according to any one of claims 1 to 6, characterized in that the measurements of the luminescence emitted by the reference compound and by the tracer compound, at wavelengths $\lambda_2$ and $\lambda_1$ respectively, are made simultaneously.

8. Use of the method according to claim 1, in a homogeneous method of detecting and/or determining an analyte in a medium in which it may be present.

9. Use according to claim 8, in a homogeneous method of detecting and/or determining an analyte in a medium in which it may be present, with the aid of an excess method consisting in:

1) adding, to said medium containing the target analyte, a first reagent made up of at least one receptor

16

for said analyte, coupled with a luminescent donor compound,
2) adding a second reagent made up of one or more other receptors for said analyte, said second reagent being coupled with a luminescent acceptor compound,
3) incubating said medium after the addition of each reagent or after the addition of both reagents,
4) exciting the resulting medium at the excitation wavelength of the luminescent donor compound,
5) measuring the signal of the luminescent donor compound at a wavelength $\lambda_1$, this measurement serving as a reference, and the signal resulting from the energy transfer at a wavelength $\lambda_2$

10. Use according to claim 8, in a homogeneous method of detecting and/or determining an analyte in a medium in which it may be present, with the aid of a competitive method consisting in:
1) adding, to said medium containing the target analyte, a first reagent made up of a receptor for said analyte, coupled with a luminescent donor compound,
2) adding a second reagent made up of the analyte coupled with a luminescent acceptor compound,
3) incubating said medium after the addition of each reagent or after the addition of both reagents,
4) exciting the resulting medium at the excitation wavelength of the luminescent donor compound,
5) measuring the signal of the luminescent donor compound at a wavelength $\lambda_1$, this measurement serving as a reference, and the signal resulting from the energy transfer at a different wavelength $\lambda_2$

11. Use according to claim 8, in a homogeneous method of detecting and/or determining an analyte in a medium in which it may be present, with the aid of a competitive method consisting in:
1) adding, to said medium containing the target analyte, a first reagent made up of a receptor for said analyte, said receptor being coupled with a luminescent acceptor compound,
2) adding a second reagent made up of the analyte coupled with a luminescent donor compound,
3) incubating said medium after the addition of each reagent or after the addition of both reagents,
4) exciting the resulting medium at the excitation wavelength of the luminescent donor compound,
5) measuring the signal of the luminescent donor compound at a wavelength $\lambda_1$, this measurement serving as a reference, and the signal resulting from the energy transfer at a different wavelength $\lambda_2$.

12. Use according to claim 8, in a homogeneous method of detecting and/or determining an analyte in a medium in which it may be present, with the aid of an method consisting in:
1) adding to said medium a first reagent made up of a receptor for said analyte,
2) adding a second reagent selected from the analyte or at least one of its receptors, one of the two reagents being coupled with a luminescent tracer compound and the other reagent containing a heavy atom or moieties containing a heavy atom, as well as a luminescent compound serving as an internal reference,
3) incubating the resulting medium either after the addition of each reagent or after the addition of both reagents,
4) exciting the resulting medium, and
5) measuring, on the one hand the signal emitted by the luminescent tracer compound, said signal being modulated by the heavy atom effect at a wavelength $\lambda_2$, and on the other hand the signal emitted by the reference compound at a wavelength $\lambda_1$.

13. Use according to any one of claims 9 to 12, characterized in that the measurements of the luminescence emitted by the reference compound and by the tracer compound, at wavelengths $\lambda_1$ and $\lambda_2$ respectively, are made simultaneously.

14. Use according to any one of claims 9 to 11, characterized in that at least one of the receptors for the analyte is bound to a solid support.

15. Method according to any one of claims 1 to 7, characterized in that correcting the measurement of the luminescence emitted by the tracer compound at wavelength $\lambda_2$ consists in fixing a counting rate on the channel measuring the luminescent emission of the reference compound at wavelength $\lambda_1$, and then, when this rate is reached, triggering the end of the measurement on the channel measuring the luminescent emission at wavelength $\lambda_2$.

FIG.1

EP 0 569 496 B1

FIG. 2

FIG. 3